# FASCICULE DE BREVET EUROPEEN

(11) **EP 3 351 504 B1**
(45) Date de publication et mention de la délivrance du brevet: **18.09.2019**
(21) Numéro de dépôt: 18152246.7
(22) Date de dépôt: 18.01.2018
(51) Int. Cl.: B81C 1/00

(54) **PROCÉDÉ DE RÉALISATION D'UNE STRUCTURE MEMS ET/OU NEMS COMPORTANT AU MOINS DEUX ÉLÉMENTS SUSPENDUS À UN SUPPORT À DES DISTANCES DIFFÉRENTES DUDIT SUPPORT**
HERSTELLUNGSVERFAHREN EINER MEMS- UND/ODER NEMS-STRUKTUR MIT WENIGSTENS ZWEI ÜBER EINEM SUBSTRAT AUFGEHÄNGTEN ELEMENTEN MIT UNTERSCHIEDLICHEN ABSTÄNDEN ZUM SUBSTRAT
METHOD OF MAKING A MEMS AND/OR NEMS STRUCTURE HAVING AT LEAST TWO ELEMENTS SUSPENDED ABOVE A SUBSTRATE AT DIFFERENT DISTANCES FROM SAID SUBSTRATE

(30) Priorité: 19.01.2017 FR 1750423
(43) Date de publication de la demande: 25.07.2018
(73) Titulaire: Commissariat à l'énergie atomique et aux énergies alternatives, 75015 Paris (FR)
(72) Inventeur: FAIN, Bruno, 38100 GRENOBLE (FR); LADNER, Carine, 38500 VOIRON (FR); ALAVA, Thomas, 38000 GRENOBLE (FR)
(74) Mandataire: Brevalex

(56) Documents cités:
- WO-A1-2015/161808
- US-A1- 2012 049 313
- PHILLIP ZELLNER ET AL: "A fabrication technology for three-dimensional micro total analysis systems", JOURNAL OF MICROMECHANICS & MICROENGINEERING, INSTITUTE OF PHYSICS PUBLISHING, BRISTOL, GB, vol. 20, no. 4, 1 avril 2010 (2010-04-01), page 45013, XP020175381, ISSN: 0960-1317

## Description

### DOMAINE TECHNIQUE ET ÉTAT DE LA TECHNIQUE ANTÉRIEURE

La présente invention se rapporte à un procédé de réalisation d'une structure microélectromécanique et/ou nanoélectromécanique comportant au moins deux éléments suspendus à un support, chaque élément étant suspendu au support à une distance différente de celle auquel l'autre élément est suspendu.

Dans le domaine des systèmes microélectromécaniques ou MEMS (Microelectromechanical systems en terminologie anglo-saxonne) et/ou nanoélectromécaniques ou NEMS (Nanoelectromechanical systems en terminologie anglo-saxonne), fréquemment un ou plusieurs éléments suspendus par rapport à un support ou substrat sont mis en oeuvre. Les éléments suspendus peuvent être mis en oeuvre dans des dispositifs fonctionnant sur le principe de la détection catharométrique, dans laquelle on mesure la conductivité par mesure de température d'un élément chauffant. Un tel élément chauffant est par exemple un fil suspendu dans une cavité fluidique contenant le gaz ou mélange gazeux à analyser.

L'élément est suspendu au-dessus d'un support formant réservoir thermique à une distance donnée. Or cette distance également appelé « gap » est une caractéristique importante dans la mesure de la conductivité thermique.

Il peut être intéressant de disposer d'un dispositif comportant plusieurs éléments suspendus à un support, la distance entre chaque élément suspendu et le support étant différente de celles séparant les autres éléments suspendus et le support.

Par example le document *"*Flow compensation in a MEMS dual-thermal conductivity detector for hydrogen sensing in natural gas", G. de Graff et al. (Transducers 2015, Anchorage, USA*)* met en oeuvre deux membranes disposées à des distances différentes par rapport à un support, afin de s'affranchir des effets de débit sur les mesures. Chaque membrane est suspendue au-dessus d'une chambre qui est séparée de l'autre chambre, les chambres présentant des profondeurs différentes.

Le procédé de fabrication mentionné dans ce document ne décrit pas comment des profondeurs différentes sont obtenues.

### EXPOSÉ DE L'INVENTION

C'est par conséquent un but de la présente invention d'offrir un procédé de fabrication d'une structure micromécanique et/ou nanomécanique, par exemple d'une structure MEMS et/ou NEMS comportant plusieurs éléments suspendus dans une même cavité, dans laquelle les éléments sont suspendus à des distances différentes du fond de la cavité.

Le but énoncé ci-dessus est atteint par un procédé de fabrication dans lequel, à partir d'un empilement d'au moins un support et d'une couche structurée comprenant les différents éléments suspendus, un masque est mis en oeuvre au-dessus de la couche structurée, le masque comprenant des ouvertures, les ouvertures présentant au moins deux tailles différentes, chaque taille étant associée à au moins un élément suspendu. Le procédé comporte en outre l'application d'une gravure anisotrope pour graver le support à des profondeurs différentes dépendantes de la taille des ouvertures et une gravure isotrope pour former au moins une cavité sous les éléments suspendus.

Grâce à l'invention, il est alors aisé de réaliser simultanément des distances entre le support et les éléments suspendus différentes.

En outre, il est possible de réaliser une structure dans laquelle les éléments sont suspendus dans une même cavité et situés des distances différentes du fond de la cavité. Pour cela le fond de la cavité est structurée en marche d'escalier ou sensiblement en plan incliné.

En d'autres termes, le procédé utilise la relation entre le taux d'ouvertures du masque et la profondeur de gravure en répartissant, dans le masque de gravure, des ouvertures de taille différentes au-dessus d'une couche structurée contenant les éléments suspendus. Il est alors possible de graver sous chaque élément suspendu sur une profondeur donnée et de fixer individuellement une distance entre un élément suspendu et le support.

Dans un exemple de réalisation, le masque comporte n zones, comportant chacune des ouvertures de taille donnée différente de celle des ouvertures des autres zones, chaque zone étant disposée au-dessus d'un élément suspendu. Ainsi on obtient un support comportant un fond comprenant des marches de hauteurs différentes dépendant de la taille des ouvertures.

Dans un autre exemple de réalisation, les éléments suspendus sont disposés les uns à côtés des autres le long d'une direction donnée, et la taille des ouvertures varie progressivement de manière monotone dans un sens ainsi le fond forme sensiblement un plan incliné.

Le procédé permet de réaliser une structure comprenant un grand nombre d'éléments suspendus sans que le procédé soit rendu plus complexe.

Le procédé selon l'invention est particulièrement adapté à la réalisation d'un dispositif d'analyse d'un mélange gazeux mettant en oeuvre la détection catharométrique et utilisant plusieurs éléments suspendus à des distances différentes du fond d'une cavité fluidique, ce qui permet de déterminer :
- dans le cas où le mélange gazeux comporte deux espèces, les concentrations des deux espèces et la pression du mélange gazeux,
- dans le cas où le mélange gazeux comportant trois espèces de pression connue, les concentrations des trois espèces du mélange gazeux.

La présente invention a alors pour objet un procédé de réalisation d'une structure microélectromécanique et/ou nanoélectromécanique comportant n éléments suspendus à un support, n étant un entier supérieur ou égal à 2, une cavité réalisée dans le support, ladite cavité présentant m profondeurs différentes dans une direction orthogonale à un plan moyen de la structure, m étant un entier supérieur ou égal à 2, comprenant :
- la réalisation d'un masque sur un empilement comportant un substrat et une couche structurée formée sur le substrat, ladite couche structurée comportant les n éléments destinés à être suspendus au-dessus de la cavité, le masque étant formé au-dessus de la couche structurée, ledit masque comportant des ouvertures de sections différentes, les ouvertures étant réparties en au moins m zones, chaque zone comportant des ouvertures de même section,
- gravure anisotrope du substrat à travers le masque et la couche structurée de sorte à définir les au moins m profondeurs dans le substrat,
- gravure isotrope du substrat pour former ladite cavité, les n éléments étant alors suspendus au-dessus de la cavité.

Par exemple, ladite cavité présente une première dimension et une deuxième dimension dans des directions orthogonales et contenu dans un plan moyen de la structure ; les ouvertures du masque de gravure sont alors par exemple réparties sensiblement en ligne et en colonne, les lignes étant alignées suivant la direction de la première dimension de la cavité et les colonnes étant alignées suivant la direction de la deuxième dimension.

Chaque zone peut comporter plusieurs colonnes successives, les sections des ouvertures étant par exemple identiques pour chaque colonne d'une même zone et différentes pour les colonnes de zones différentes, de sorte à former une cavité comportant un fond présentant des marches de profondeur différentes, chaque marche correspondant à une zone.

En variante, chaque zone comporte une colonne, la section des ouvertures de chaque colonne variant de manière monotone au moins le long la direction de la première dimension de sorte à former une cavité munie d'un fond formant sensiblement un plan incliné.

Avantageusement, la gravure isotrope est une gravure ionique réactive.

De préférence, chaque zone est formée au moins à l'aplomb d'un élément de la couche structurée et les ouvertures de chaque zone sont formées au moins à l'aplomb des espaces entre deux éléments de la couche structurée.

Les éléments destinés à être suspendus peuvent être des membranes munies de trous, des ouvertures de chaque zone du masque de gravure étant également formées à l'aplomb des trous des membranes.

L'empilement peut comporter une couche sacrificielle entre la couche structurée et le substrat, le procédé peut alors comporter en outre une étape de retrait de la couche sacrificielle pour désolidariser les éléments suspendus de la couche sacrificielle, par exemple au moyen d'acide fluorhydrique.

La couche structurée est par exemple en Si ou en SiN + Pt.

De préférence, l'empilement est réalisé à partir d'un substrat SOI et la couche structurée est obtenue par structuration de la couche de silicium monocristallin.

Le procédé de réalisation comporte avantageusement l'étape de scellement d'un capot après la libération des éléments suspendus, au-dessus de la cavité et en regard des éléments suspendus.

Par exemple, n est égal à m et chaque élément suspendu est suspendu au-dessus de la cavité à une distance différente d'un fond de celle-ci.

Le procédé de réalisation peut en outre comporter:
- la réalisation de connexions électriques au niveau des éléments suspendus,
- réalisation de moyens de chauffage des éléments suspendus,
- connexion des éléments suspendus à des moyens de polarisation et à des moyens de mesure de la résistance électrique des éléments suspendus.

### BRÈVE DESCRIPTION DES DESSINS

La présente invention sera mieux comprise sur la base de la description qui va suivre et des dessins en annexe sur lesquels:
- les figures 1A à 1R sont des représentations schématiques d'éléments obtenus lors d'étapes d'un exemple du procédé de réalisation selon l'invention,
- les figures 2A et 2B sont des vues de dessus des éléments des figures 1J et 1J',
- la figure 3 est une vue en coupe longitudinale schématique d'un exemple de réalisation d'une structure pouvant être obtenue par le procédé selon l'invention et le masque 2B,
- la figure 4 est une vue en coupe longitudinale schématique d'un autre exemple de réalisation d'une structure pouvant être obtenue par le procédé selon l'invention et le masque 2A,
- les figures 5A à 5D sont des vues de dessus de différents exemples d'éléments suspendus pouvant être mis en oeuvre par le présent procédé,

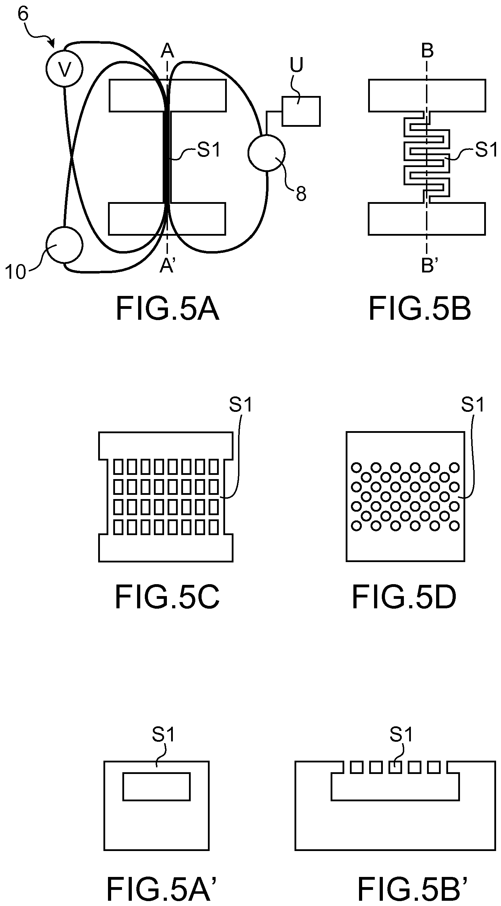

- les figures 5A' et 5B' sont des vues en coupe transversale selon le plan A-A' et B-B' respectivement des figures 5A et 5B.

### EXPOSÉ DÉTAILLÉ DE MODES DE RÉALISATION PARTICULIERS

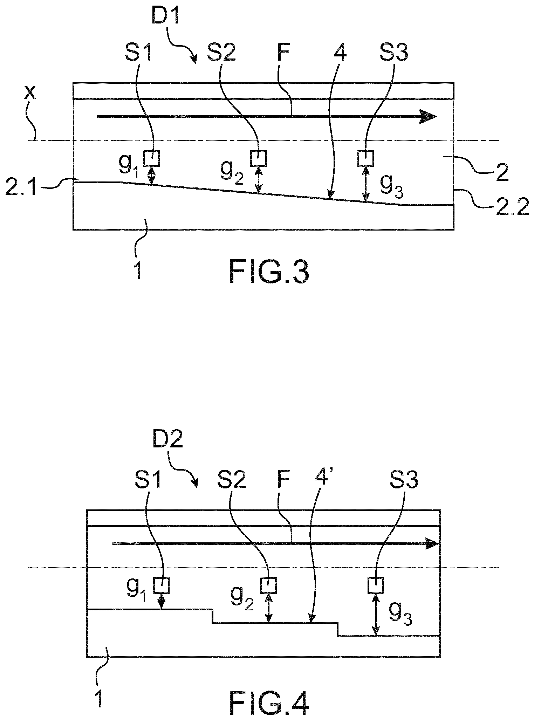

Sur les figures 3 et 4, on peut voir des exemples de structures pouvant être obtenues avec le procédé selon l'invention.

Sur la figure 3, la structure D1 comporte un support 1 et au moins un canal 2 réalisé dans ce support 1. Le support est par exemple un substrat, par exemple en semi-conducteur ou en verre. Le canal comporte un fond 4 et des parois latérales.

La structure comporte également trois éléments de mesure S1, S2, S3 disposés l'un à côté de l'autre dans la direction F.

Le plan moyen de la structure est le plan contenant les éléments suspendus.

Dans l'exemple représenté, les éléments de mesure S1, S2, S3 sont disposés parallèlement les uns par rapport aux autre et sont suspendus sensiblement perpendiculairement à la direction du flux F.

Les éléments suspendus peuvent présenter des formes très variées. Sur les figures 5A à 5D, on peut voir différents exemples de réalisation d'un seul élément de mesure vu de dessus et sur les figures 5A' et 5B' on peut voir des vues en coupe selon le plan A-A' et B-B' des figures 5A et 5B respectivement, les vues en coupe des figures 5C et 5D seraient similaires à la figure 5B'.

Sur la figure 5A, l'élément de mesure est un fil droit, sur la figure 5B il s'agit d'un fil en en forme de serpentin, sur les figures 5C et 5D, les éléments de mesure sont des membranes suspendus ajourées. En variante, les membranes pourraient être pleines. De préférence tous les éléments de mesure d'un capteur sont de structure identique ou similaire.

Il peut être envisagé de fonctionnaliser la surface des éléments de mesure. Dans ce cas les propriétés des éléments de mesure sont modifiées par les espèces chimiques en présence dans la phase gazeuse, par exemple la masse.

L'élément de mesure S1 est à une distance g1 du fond du canal, l'élément de mesure S2 est à une distance g2 du fond du canal et l'élément de mesure S3 est à une distance g3 du fond du canal.

Les distances g1, g2 et g3 sont différentes. Dans l'exemple représenté g1 est inférieure à g2, qui est elle-même inférieure à g3.

Dans l'exemple représenté, le fond 4 du canal est incliné vers le bas dans le sens F et est contenu dans un plan.

Sur la figure 4, la structure D2 comporte un fond 4' en forme de marche d'escalier et est donc contenu dans plusieurs plans parallèles entre eux.

Dans les exemples décrits, la distance entre l'élément suspendu et le fond du canal varie de manière monotone. Mais le procédé selon l'invention permet d'envisager que g1 et g3 soient supérieures à g2 et g3 supérieure à g1.

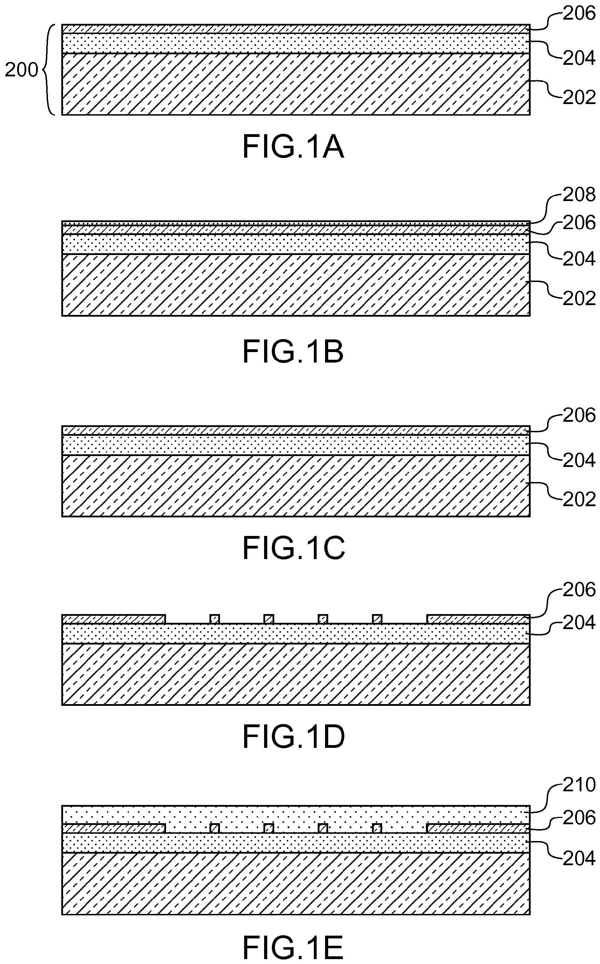

Un exemple du procédé de réalisation selon l'invention d'un dispositif d'analyse similaire à celui de la figure 4 comportant deux éléments de mesure selon l'invention va maintenant être décrit à l'aide des figures 1A à 1R.

On utilise par exemple un substrat 200 silicium sur isolant ou SOI (Silicon On Insulate en terminologie anglo-saxonne). Le substrat est représenté sur la figure 1A. Le substrat 200 comporte un substrat support en silicium 202, une couche d'oxyde de silicium 204 et une couche de silicium monocristallin 206 sur la couche d'oxyde 204. En variante, le substrat peut être un substrat silicium Si oxydé sur lequel une couche de Si amorphe ou polycristallin est formée.

Lors d'une étape suivante, on forme une couche d'oxyde 208 sur la couche 206, puis on réalise une implantation d'ions Bore. L'élément ainsi obtenu est représenté sur la figure1B.

Lors d'une étape suivante, la couche d'oxyde est retirée par exemple par gravure chimique à base d'acide fluorhydrique (HF). L'élément ainsi obtenu est représenté sur la figure 1C.

Lors d'une étape suivante, la couche de silicium 206 est structurée par exemple par photolithographie et gravure afin de former les éléments de mesure.

L'élément ainsi obtenu est représenté sur la figure 1D.

Lors d'une étape suivante, une couche protectrice 210 est formée sur les éléments sensibles, il s'agit par exemple d'une couche d'oxyde, d'épaisseur par exemple 500 nm. La couche 210 est par exemple déposée par une technique de dépôt chimique en phase vapeur. La couche 210 est ensuite planarisée.

L'élément ainsi obtenu est représenté sur la figure 1E.

Lors d'une étape suivante, la couche 210 est structurée pour former des accès 212 à la couche 206 en dehors de la partie comprenant les éléments de mesure. Cette structuration est réalisée par photolithographie et gravure.

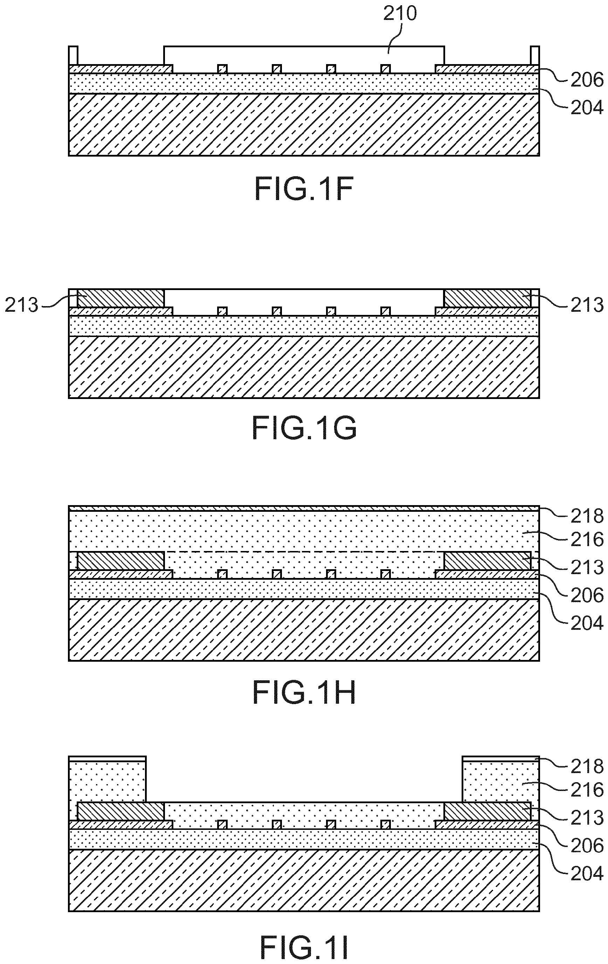

L'élément ainsi obtenu est représenté sur la figure 1F.

Dans une étape suivante, on réalise les plots de contact 213 au niveau des accès 212. Une couche métallique 214, par exemple du AISi est formée sur la couche 210, par exemple par dépôt, et est ensuite structurée par exemple par gravure.

L'élément ainsi obtenu est représenté sur la figure 1G.

Lors d'une étape suivante une autre couche d'oxyde 216 est formée sur la couche 210 et sur les plots de contact 213, par exemple par dépôt. Elle présente par exemple une épaisseur de 1 µm. Une couche de Si amorphe 218 est ensuite formée sur la couche 216, d'épaisseur par exemple de 100 nm. La couche 218 peut être formée en tout matériau qui est résistant au HF et qui peut être gravé par photo-lithogravure, par exemple du nitrure de bore.

L'élément ainsi obtenu est représenté sur la figure 1H.

Lors d'une étape suivante, les couches 216 et 218 sont structurées pour dégager la partie active du capteur, cette structuration est par exemple réalisée par photolithographie et gravure.

L'élément ainsi obtenu est représenté sur la figure 1I.

Les éléments obtenus avec les étapes qui suivent sont représentés dans deux plans de coupe au niveau de chaque élément de mesure.

Lors d'une étape suivante, une couche d'oxyde 220 est déposée. Ensuite une couche de résine 221 est déposée, celle-ci est destinée à former un masque.

Lors d'une étape suivante, la couche 221 est structurée par photolithographie de sorte à former des ouvertures dans la couche 221.

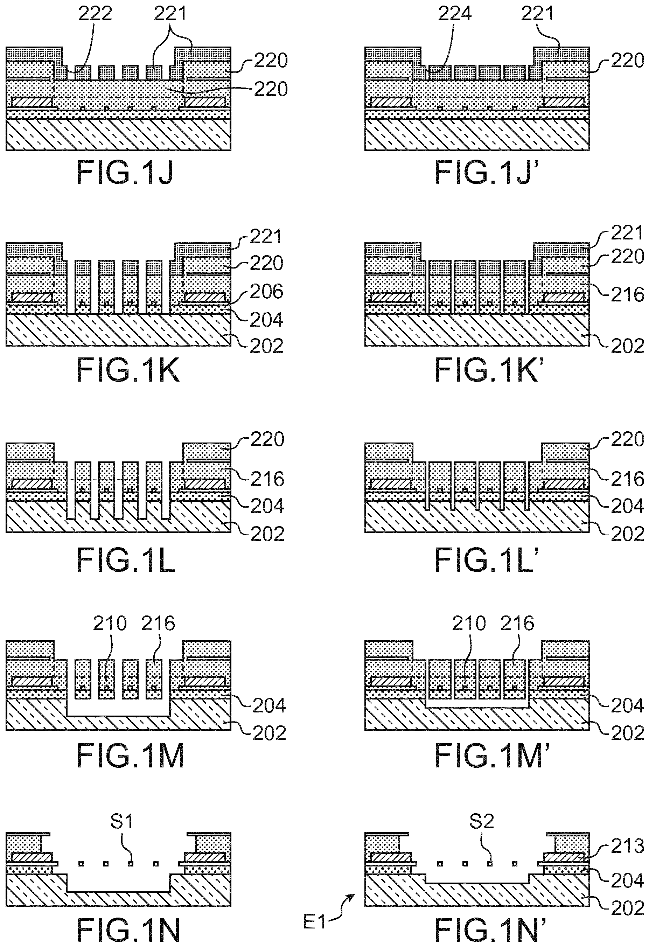

L'étape de photolithographie est telle que des premières ouvertures 222 présentant une première section sont réalisées dans une zone A1 de la couche 221 au droit du premier élément de mesure S1 (figure 1J) et des deuxièmes ouvertures 224 présentant une deuxième section sont réalisées dans une zone de la couche 220 au droit du premier élément de mesure S2 (figure 1J'). La première section et la deuxième section sont différentes. Dans l'exemple représenté, la première section est supérieure à la deuxième section.

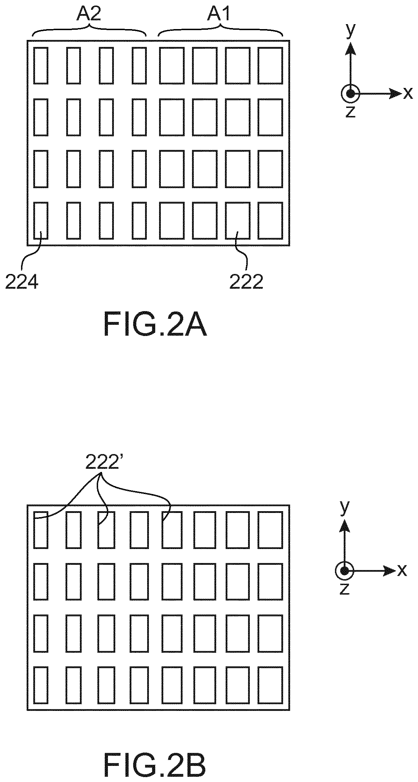

Sur la figure 2A, on peut voir l'élément des figures 1J et 1J' vu de dessus représenté de manière schématique. On distingue les deux zones A1, A2 du masque 221 avec des ouvertures 222 et 224 de sections différentes. Ce masque 221 permet de réaliser une structure comportant deux marches tel que représenté sur la figure 4.

Le masque 221 comporte des colonnes d'ouvertures disposées les unes à côté des autres. Dans l'exemple représenté plusieurs colonnes ont des ouvertures de même section. Les colonnes s'étendent sur toute la largeur des éléments suspendus dans la direction Y.

La section des ouvertures dans le masque détermine la profondeur de gravure comme cela sera expliqué par la suite. En réalisant deux zones avec chacune comportant des ouvertures de section donnée, il est possible de réaliser des gravures de deux profondeurs différentes.

Le masque formé dans la couche d'oxyde a également pour fonction de permet de structurer les différentes couches autour des éléments de mesure.

Pour réaliser la structure D2, le masque comporte trois zones avec des ouvertures de sections différentes.

Sur la figure 2B, on peut voir un autre exemple de réalisation d'un masque dans lequel la section des ouvertures varie progressivement de manière monotone le long de la direction X, et permettant de réaliser un fond incliné sensiblement plan tel que représenté sur la figure 3. Dans l'exemple représenté, la sections des ouvertures 222' varie d'une colonne à l'autre et est croissante de la gauche vers la droite dans la représentation de la figure 2B.

La variation de la section des ouvertures est très progressive d'une colonne d'ouvertures à l'autre et les ouvertures sont très proches les unes des autres afin d'assurer une variation quasi-continue de la profondeur de gravure et limiter l'apparition de marche

En variante encore, les deux masques des figures 2A et 2B pourraient être combinés afin de réaliser une cavité dont le fond comporterait des paliers et des parties inclinées.

Dans les exemples des figures 2A et 2B, les ouvertures sont de forme rectangulaire mais ceci n'est pas limitatif, elles pourraient être de forme carrées, ronde...

Lors d'une étape suivante, on réalise une gravure jusqu'à atteindre le substrat 200, par exemple par gravure anisotrope. Des ouvertures atteignant le substrat 200 ayant la première section sont ainsi formées dans la première zone et des ouvertures atteignant le substrat 202 ayant la deuxième section sont ainsi formées dans la deuxième zone.

L'élément ainsi obtenu est représenté sur les figures 1K, 1K'.

Lors d'une étape suivante, on réalise une gravure anisotrope du substrat par exemple par gravure ionique réactive profonde ou DRIE (pour Deep Reactive Ion Etching en terminologie anglo-saxonne). Du fait des ouvertures de section plus grande dans la première zone A1 que dans la deuxième zone A2, la gravure dans le substrat 202 est plus profonde sous la première zone A1 que sous la deuxième zone A2. Le substrat comporte alors une pluralité de cavités dont la profondeur dépend de la section de l'ouverture qui a permis sa gravure.

L'élément ainsi obtenu est représenté sur les figures 1L et 1L'.

Lors d'une étape suivante, le substrat 202 est structuré pour réunir les ouvertures dans la première zone et d'autre les ouvertures dans la deuxième zone. Cette structuration est par exemple réalisée par gravure isotrope. La gravure grave les parois latérales des cavités qui sont alors réunies en une seule cavité. La gravure isotrope a également pour effet de graver le substrat vers l'extérieur sous les plots de contact, l'épaisseur de matériau entre les cavités est alors telle que, lors de la gravure isotrope, toute cette épaisseur est gravée avant qu'une gravure trop importante au niveau des plots de contact n'ait lieu. Les écartements des ouvertures dans le masque sont alors choisis en conséquence. La gravure isotrope est par exemple une gravure ionique réactive, par exemple réalisée en utilisant un gaz, par exemple du SF₆.

Dans cet exemple, en tenant compte de la quantité d'oxyde encapsulant les nanostructures en Si et du rapport de la sélectivité de la gravure Si isotrope vis-à-vis du SiO₂, qui est environ de 1000, la distance entre les ouvertures peut atteindre 50µm.

La gravure ionique réactive peut être isotrope ou anisotrope en fonction des conditions d'utilisation de l'équipement, notamment de la tension de polarisation appliquée au substrat.

L'élément ainsi obtenu est représenté sur les figures 1M, 1M' et correspond à la structure selon l'invention.

Lors d'une étape suivante, l'oxyde des couches 204, 210 et 216 autour des éléments suspendus est retiré par exemple par de l'acide fluorhydrique.

L'élément E1 ainsi obtenu est représenté sur la figure 1N et 1N'.

Il peut être prévus suivant l'utilisation faite de la structure des figures 1N et 1N' de lui ajouter un capot pour former une cavité fermée et/ou protéger les éléments suspendus.

Un exemple de réalisation d'un tel capot et d'assemblage avec la structure des figures 1N et 1N' va maintenant être décrit.

Un capot est fabriqué par ailleurs par exemple à partir d'un substrat 300 en silicium, un autre matériau semi-conducteur, voire un substrat, pourrait convenir.

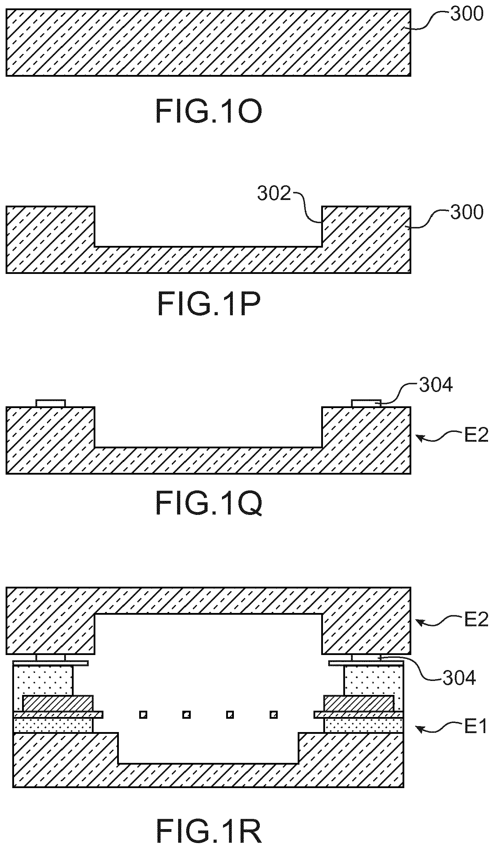

Le substrat 300 est représenté sur la figure 1O.

Une ouverture ou évidement 302 est réalisé en face avant du substrat, par exemple de 100 µm de profondeur, par exemple par photolithographie et gravure.

L'élément ainsi obtenu est représenté sur la figure 1P.

Lors d'une étape suivante, un cordon de scellement 304 est formé en face avant de sorte à entourer l'évidement. Le cordon est par exemple en matériau polymère et est par exemple déposé.

L'élément E2 ainsi obtenu est représenté sur la figure 1Q.

Lors d'une étape suivante, l'élément E1 est scellé sur l'élément E2 de sorte que l'évidement soit en regard des éléments de mesure. Le scellement est par exemple réalisé par thermocompression.

D'autres types de scellement métalliques avec cordons permettant une thermocompression Au/Au, eutectique Au/Si pourraient être envisagés.

L'élément ainsi obtenu est représenté sur la figure 1R et forme le dispositif d'analyse. Les plots de contacts sont destinés à être reliés à un circuit électronique comportant à la fois les moyens de polarisation des éléments de mesure, de mesure des résistances électriques des éléments de mesure et de traitement des mesures pour déterminer la concentration en espèces gazeuses et éventuellement la pression du mélange.

Il sera compris que le procédé de réalisation selon l'invention s'applique à la réalisation d'une structure à n éléments suspendus, chacun suspendu à une distance différente du support, n étant un entier supérieur ou égal 2. Le procédé permet avantageusement la réalisation simultanée d'un grand nombre d'éléments suspendus sans que le procédé soit rendu plus complexe.

En variante, les sections d'ouvertures dans le masque pourraient être réparties de sorte que la profondeur du fond augmente de manière non monotone. Par exemple, il pourrait être prévue quatre zones d'ouvertures, une première zone avec des ouvertures de section s1, une deuxième zone avec des ouvertures de section s2, une troisièmes zone avec des ouvertures de sections 3 et une quatrième zone avec des ouvertures de section s4. Les zones seraient disposées les unes à côté des autres le long de la direction X de la première zone à la quatrième zone, la section s1 étant inférieure à s2, s2 est inférieure à s3 et s3 étant supérieure à s4.

De même dans le cas d'ouvertures dont la section varie progressivement, on pourrait prévoir que la variation progressive ne soit pas monotone. Le fond de la cavité comporterait alors des pentes d'orientations opposées.

En variante encore, une variation de profondeur dans la direction Y associée ou non à une variation de profondeur dans la direction X ne sort pas du cadre de la présente invention.

Dans l'exemple de procédé décrit ci-dessus, les éléments suspendus sont tous suspendus dans la même cavité, mais on pourrait envisager qu'ils soient suspendus dans des cavités distinctes et de réaliser simultanément des cavités de profondeurs différentes, chaque cavité pouvant elle-même présentés plusieurs profondeurs.

Il sera compris également que, si la réalisation de profondeurs différentes sous les éléments suspendus a été décrite en détail, les étapes de gravures isotrope et anisotrope agissent également sur les zones entre les éléments suspendus.

La distance entre les colonnes d'ouvertures est fixée par la disposition et la forme des éléments suspendus.

Dans le cas d'éléments suspendus formés par des membranes comportant des trous, les ouvertures des masques sont alignées avec les espaces entre les membranes et avec les trous dans les membranes.

Il sera également compris que plusieurs éléments suspendus successifs pourraient être à une même distance du fond de la cavité, par exemple le fond de la cavité comporterait des paliers s'étendant sous plusieurs éléments suspendus.

Le procédé de réalisation selon l'invention est particulièrement adapté à la réalisation d'un dispositif d'analyse d'un mélange gazeux mettant en oeuvre la détection catharométrique comportant plusieurs éléments suspendus à des distances différentes d'un support formant un réservoir thermique. La cavité forme un canal alimenté avec le mélange gazeux à analyser.

Le dispositif comporte également des moyens de polarisation 6 des éléments suspendus, des moyens de chauffage 10 des éléments suspendus et des moyens de mesure 8 de la résistance électrique des éléments suspendus (figure 5A).

Les résistances électriques des éléments suspendus sont mesurées.

En connaissant pour chaque élément suspendu, la fonction reliant la conductivité thermique à la résistance électrique mesurée il est possible de remonter à la composition du mélange gazeux.

En mettant en oeuvre des éléments suspendus à différents hauteurs et en tenant compte du libre parcours moyen du mélange gazeux qui dépend de la distance entre les élément suspendus et le support, il est possible de déterminer la composition d'un mélange de deux espèces et sa pression et de déterminer la composition d'un mélange de trois espèces connaissant la pression du mélange gazeux.

## Revendications

1. Procédé de réalisation d'une structure microélectromécanique et/ou nanoélectromécanique (D1) comportant n éléments suspendus (S1, S2, S3) à un support (1), n étant un entier supérieur ou égal à 2, une cavité réalisée dans le support (1), ladite cavité présentant m profondeurs différentes dans une direction orthogonale à un plan moyen de la structure, m étant un entier supérieur ou égal à 2, comprenant :
- la réalisation d'un masque (221) sur un empilement comportant un substrat (200) et une couche structurée (206) formée sur le substrat, ladite couche structurée (206) comportant les n éléments (S1, S2, S3) destinés à être suspendus au-dessus de la cavité, le masque (221)étant formé au-dessus de la couche structurée (206), ledit masque (221) comportant des ouvertures (222) de sections différentes, les ouvertures (222) étant réparties en au moins m zones (A1, A2), chaque zone (A1, A2) comportant des ouvertures (222) de même section,
- gravure anisotrope du substrat (206) à travers le masque et la couche structurée (206) pour former au moins m zones gravées à des profondeurs différentes, de sorte à définir les au moins m profondeurs dans le substrat,
- gravure isotrope du substrat (206) pour former ladite cavité réunissant les au moins m zones gravées, les n éléments (S1, S2, S3) étant alors suspendus au-dessus de la cavité.

2. Procédé de réalisation selon la revendication 1, dans lequel ladite cavité présente une première dimension et une deuxième dimension dans des directions orthogonales et contenues dans un plan moyen de la structure, et dans lequel les ouvertures (222) du masque de gravure (221) sont réparties sensiblement en ligne et en colonne, les lignes étant alignées suivant la direction de la première dimension de la cavité et les colonnes étant alignées suivant la direction de la deuxième dimension.

3. Procédé de réalisation selon la revendication 2, dans lequel chaque zone (A1, A2) comporte plusieurs colonnes successives, les sections des ouvertures étant identiques pour chaque colonne d'une même zone et différentes pour les colonnes de zones différentes, de sorte à former une cavité comportant un fond présentant des marches de profondeur différentes, chaque marche correspondant à une zone.

4. Procédé de réalisation selon la revendication 2, dans lequel chaque zone comporte une colonne, la section des ouvertures de chaque colonne variant de manière monotone au moins le long la direction de la première dimension de sorte à former une cavité munie d'un fond formant sensiblement un plan incliné.

5. Procédé de réalisation selon l'une des revendications 1 à 4, dans lequel la gravure isotrope est une gravure ionique réactive.

6. Procédé de réalisation selon l'une des revendications 1 à 5, dans lequel chaque zone est formée au moins à l'aplomb d'un élément de la couche structurée (206) et les ouvertures (222) de chaque zone sont formées au moins à l'aplomb des espaces entre deux éléments de la couche structurée (206).

7. Procédé de réalisation selon la revendication 6, dans lequel les éléments destinés à être suspendus sont des membranes munies de trous, des ouvertures de chaque zone du masque de gravure étant également formées à l'aplomb des trous des membranes.

8. Procédé de réalisation selon l'une des revendications 1 à 7, dans lequel l'empilement comportant une couche sacrificielle entre la couche structurée et le substrat, le procédé comporte en outre une étape de retrait de la couche sacrificielle pour désolidariser les éléments suspendus de la couche sacrificielle, par exemple au moyen d'acide fluorhydrique.

9. Procédé de réalisation selon l'une des revendications 1 à 8, dans lequel la couche structurée est en Si ou en SiN + Pt.

10. Procédé de réalisation selon la revendication 9, dans lequel l'empilement est réalisé à partir d'un substrat SOI et dans lequel la couche structurée(206) est obtenue par structuration de la couche de silicium monocristallin.

11. Procédé de réalisation selon l'une des revendications 1 à 10, comportant l'étape de scellement d'un capot (E2) après la libération des éléments suspendus, au-dessus de la cavité et en regard des éléments suspendus.

12. Procédé de réalisation selon l'une des revendications 1 à 11, dans lequel n est égal à m et chaque élément suspendu est suspendu au-dessus de la cavité à une distance différente d'un fond de celle-ci.

13. Procédé de réalisation d'un dispositif d'analyse d'un mélange gazeux mettant en oeuvre le procédé selon l'une des revendications 1 à 12, et comportant :
- la réalisation de connexions électriques au niveau des éléments suspendus,
- réalisation de moyens de chauffage des éléments suspendus,
- connexion des éléments suspendus à des moyens de polarisation et à des moyens de mesure de la résistance électrique des éléments suspendus.

## Patentansprüche

1. Verfahren zur Herstellung einer mikroelektromechanischen und/oder nanoelektromechanischen Struktur (D1), die n Elemente (S1, S2, S3) enthält, die an einem Träger (1) abgehängt sind, wobei n eine ganze Zahl größer oder gleich 2 ist, einen Hohlraum, der in dem Träger (1) ausgebildet ist, wobei der Hohlraum m verschiedene Tiefen in einer Richtung orthogonal zu einer Mittelebene der Struktur aufweist, wobei m eine ganze Zahl größer oder gleich 2 ist, umfassend:
- Herstellen einer Maske (211) auf einer Stapelung, die ein Substrat (200) und eine auf dem Substrat gebildete strukturierte Schicht (206) enthält, wobei die strukturierte Schicht (206) die n Elemente (S1, S2, S3) enthält, die dazu bestimmt sind, oberhalb des Hohlraums abgehängt zu werden, wobei die Maske (221) oberhalb der strukturierten Schicht (206) ausgebildet wird, wobei die Maske (221) Öffnungen (222) mit verschiedenen Querschnitten aufweist, wobei die Öffnungen (222) in zumindest m Zonen (A1, A2) verteilt sind und jede Zone (A1, A2) Öffnungen (222) mit dem gleichen Querschnitt aufweist,
- anisotropes Ätzen des Substrats (206) durch die Maske und die strukturierte Schicht (206) hindurch, um zumindest m geätzte Zonen mit verschiedenen Tiefen zu bilden, so dass die zumindest m Tiefen im Substrat definiert werden,
- isotropes Ätzen des Substrats (206), um den Hohlraum zu bilden, der die zumindest m geätzten Zonen verbindet, wobei somit die n Elemente (S1, S2, S3) oberhalb des Hohlraums abgehängt werden.

2. Herstellungsverfahren nach Anspruch 1, wobei der Hohlraum eine erste Abmessung und eine zweite Abmessung in orthogonalen Richtungen und in einer Mittelebene der Struktur aufweist und wobei die Öffnungen (222) der Ätzmaske (221) im Wesentlichen in Zeilen und Spalten verteilt sind, wobei die Zeilen in der Richtung der ersten Abmessung des Hohlraums ausgerichtet sind und die Spalten in der Richtung der zweiten Abmessung ausgerichtet sind.

3. Herstellungsverfahren nach Anspruch 2, wobei jede Zone (A1, A2) mehrere aufeinanderfolgende Spalten enthält, wobei die Querschnitte der Öffnungen bei jeder Spalte derselben Zone identisch sind und bei den Spalten verschiedener Zonen unterschiedlich sind, so dass ein Hohlraum mit einem Boden gebildet wird, der unterschiedlich tiefe Stufen enthält, wobei jede Stufe einer Zone entspricht.

4. Herstellungsverfahren nach Anspruch 2, wobei jede Zone eine Spalte enthält, wobei der Querschnitt der Öffnungen einer jeden Spalte zumindest in der Richtung der ersten Abmessung monoton variiert, so dass ein Hohlraum gebildet wird, der mit einem Boden versehen ist, der im Wesentlichen eine geneigte Ebene bildet.

5. Herstellungsverfahren nach einem der Ansprüche 1 bis 4, wobei das isotrope Ätzen ein reaktives lonenätzen ist.

6. Herstellungsverfahren nach einem der Ansprüche 1 bis 5, wobei jede Zone zumindest lotrecht zu einem Element der strukturierten Schicht (206) ausgebildet ist und die Öffnungen (222) jeder Zone zumindest lotrecht zu den Räumen zwischen zwei Elementen der strukturierten Schicht (206) ausgebildet sind.

7. Herstellungsverfahren nach Anspruch 6, wobei die abzuhängenden Elemente Membrane mit Lochungen sind, wobei die Öffnungen einer jeden Zone der Ätzmaske auch lotrecht zu den Lochungen der Membrane ausgebildet sind.

8. Herstellungsverfahren nach einem der Ansprüche 1 bis 7, wobei bei der Stapelung mit einer Opferschicht zwischen der strukturierten Schicht und dem Substrat das Verfahren ferner einen Schritt des Entfernens der Opferschicht umfasst, um die abgehängten Elemente von der Opferschicht zu trennen, beispielsweise mittels Flusssäure.

9. Herstellungsverfahren nach einem der Ansprüche 1 bis 8, wobei die strukturierte Schicht aus Si oder aus SiN + Pt besteht.

10. Herstellungsverfahren nach Anspruch 9, wobei die Stapelung ausgehend von einem SOI-Substrat gebildet wird und die strukturierte Schicht (206) durch Strukturierung der Schicht aus monokristallinem Silizium erhalten wird.

11. Herstellungsverfahren nach einem der Ansprüche 1 bis 10, umfassend nach dem Freigeben der abgehängten Elemente den Schritt des Versiegelns einer Abdeckung (E2) oberhalb des Hohlraums und gegenüber den abgehängten Elementen.

12. Herstellungsverfahren nach einem der Ansprüche 1 bis 11, wobei n gleich m ist und jedes abgehängte Element oberhalb des Hohlraums in einem unterschiedlichen Abstand von einem Boden desselben abgehängt ist.

13. Verfahren zum Herstellen einer Vorrichtung zur Analyse eines Gasgemischs, bei dem das Verfahren nach einem der Ansprüche 1 bis 12 durchgeführt wird, und umfassend:
- Herstellen von elektrischen Verbindungen an den abgehängten Elementen,
- Ausbilden von Mitteln zum Erwärmen der abgehängten Elemente,
- Verbinden der abgehängten Elemente mit Polarisationsmitteln und mit Mitteln zum Messen des elektrischen Widerstands der abgehängten Elemente.

## Claims

1. Method of fabricating a microelectromechanical and/or nanoelectromechanical structure (D1) comprising n elements suspended (S1, S2, S3) from a support (1), n being an integer greater than or equal 2, a cavity made in the support (1), said cavity having m different depths along a direction orthogonal to a median plane of the structure, m being an integer greater than or equal 2, comprising:
- fabrication of a mask (221) on a stack comprising a substrate (200) and a structured layer (206) formed on the substrate, said structured layer (206) comprising the n elements (S1, S2, S3) that will be suspended above the cavity, the mask (221) being formed above the structured layer (206), said mask (221) comprising openings (222) with different sections, the openings (222) being distributed in at least m zones (A1, A2), each zone (A1, A2) comprising openings with the same section,
- anisotropic etching of the substrate (206) through the mask and the structured layer (206) to make the at least m zones at different depths so as to define at least m depths in the substrate,
- isotropic etching of the substrate (206) to form said cavity connecting the at least m etched zones, the n elements (S1, S2, S3) then being suspended above the cavity.

2. Fabrication method according to claim 1, in which said cavity has a first dimension and a second dimension along orthogonal directions and contained in a median plane of the structure, and in which the openings -222) in the etching mask (221) are distributed approximately in rows and columns, the rows being aligned along the direction of the first dimension of the cavity and the columns being aligned along the direction of the second dimension.

3. Fabrication method according to claim 2, in which each zone (A1, A2) comprises several successive columns, the sections of the openings being identical for each column in the same zone and different for columns in different zones, so as to form a cavity comprising a bottom with steps of different depths, each step corresponding to a zone.

4. Fabrication method according to claim 2, in which each zone comprises a column, the section of the openings in each column varying monotonously at least along the direction of the first dimension so as to form a cavity with a bottom approximately forming an inclined plane.

5. Fabrication method according to one of the claims 1 to 4, in which the isotropic etching is a reactive ion etching.

6. Fabrication method according to one of the claims 1 to 5, in which each zone is formed at least vertically in line with one element of the structured layer (206) and the openings (222) in each zone are formed at least vertically in line with spaces between two elements of the structured layer (206).

7. Fabrication method according to claim 6, in which the elements that will be suspended are membranes provided with holes, openings in each zone of the etching mask also being formed vertically in line with holes in the membranes.

8. Fabrication method according to one of the claims 1 to 7, the stack comprising a sacrificial layer between the structured layer and the substrate, in which the method also comprises a step in which the sacrificial layer is removed to release suspended elements from the sacrificial layer, for example by means of hydrofluoric acid.

9. Fabrication method according to one of the claims 1 to 8, in which the structure layer is made of Si or SiN + Pt.

10. Fabrication method according to claim 9, in which the stack is made from an SOI substrate and in which the structured layer is obtained by structuring of the monocrystalline silicon layer.

11. Fabrication method according to one of the claims 1 to 10, comprising the step to bond a cap (E2) after the suspended elements have been released, above the cavity and facing the suspended elements.

12. Fabrication method according to one of the claims 1 to 11, in which n is equal to m and each suspended element is suspended above the cavity at a different distance from the bottom of the cavity.

13. Fabrication method for fabricating a device for analysis of a gas mix making use of the method according to one of the claims 1 to 12, and comprising:
- fabrication of electrical connections at the suspended elements,
- fabrication of heating means for the suspended elements,
- connection of suspended elements to polarization means and to means for measuring the electrical resistance of the suspended elements.
